# EUROPEAN PATENT APPLICATION

(11) **EP 4 550 310 A1**
(43) Date of publication of application: **07.05.2025**
(21) Application number: 23831365.4
(22) Date of filing: 26.06.2023
(51) Int. Cl.: G10K 15/04, A61M 21/00

(54) **SIGNAL PROCESSING DEVICE, CONGNITIVE FUNCTION IMPROVEMENT SYSTEM, SIGNAL PROCESSING METHOD, AND PROGRAM**

(30) Priority: 28.06.2022 JP 2022103165
(71) Applicant: Pixie Dust Technologies, Inc., Tokyo, 104-0028 (JP); Shionogi & Co., Ltd, Osaka-shi, Osaka 541-0045 (JP)
(72) Inventor: NAGATANI Yoshiki, Tokyo 104-0028 (JP); TAKAZAWA Kazuki, Tokyo 104-0028 (JP); KATAYAMA Haruki, Tokyo 104-0028 (JP)
(74) Representative: Plasseraud IP
(86) International application number: PCT/JP2023/023523
(87) International publication number: WO 2024/004924

(57) **Abstract**

A signal processing apparatus of one embodiment of the present disclosure has a receiving means for receiving an input audio signal, and a generating means for generating a modulated audio signal having an amplitude change corresponding to a frequency of gamma waves by performing a modulation process including amplitude modulation on the input audio signal received by the receiving means, and an output means for outputting a content and modulation information in association with each other, the content including the modulated audio signal generated by the generation means, the modulation information being related to the modulation process performed to generate the modulated audio signal

## Description

### [Technical field]

The present disclosure relates to a signal processing apparatus, a cognitive function improvement system, a signal processing method, and a program.

### [Background Art]

There is a research report showing that inducing gamma waves in the brain of an organism by subjecting it to pulsating sound stimuli at a frequency of about 40 times per second is effective in improving the organism's cognitive function (see non-patent document 1).

Gamma waves refer to neural oscillations that capture periodic neural activity in the brain's cortex using electrophysiological techniques such as electroencephalography and magnetoencephalography, and whose frequencies fall within the gamma band (25 to 140 Hz).

Patent Document 1 discloses adjusting the volume by increasing or decreasing the amplitude of a sound wave or sound track to create a rhythmic stimulus that corresponds to a stimulation frequency that induces brainwave entrainment.

### [Prior Art Documents]

### [Patent Documents]

[Patent Document 1] JP 2020-501853

### [Non-patent Document]

[Non-Patent Document 1] Multi-sensory Gamma Stimulation Ameliorates Alzheimer's-Associated Pathology and Improves Cognition Cell 2019 Apr 4; 177(2):256-271.e22. doi: 10.1016/j.cell.2019.02.014.

### [Summary of the Invention]

### [Technical Problem]

When playing back a content that includes an audio signal, if it is unclear whether the content includes an amplitude-modulated audio signal, and if so, what type of amplitude modulation it is, this may cause inconvenience to the user. For example, a user hoping to improve cognitive function may find it difficult to determine which content to listen to or watch and for how long.

An object of the present disclosure is to provide a technique for improving user convenience in determining which content to play back or in determining the listening or viewing manner of the content.

### [Solution to Problem]

A signal processing apparatus of one embodiment of the present disclosure has a receiving means for receiving an input audio signal, and a generating means for generating a modulated audio signal having an amplitude change corresponding to a frequency of gamma waves by performing a modulation process including amplitude modulation on the input audio signal received by the receiving means, and an output means for outputting a content and modulation information in association with each other, the content including the modulated audio signal generated by the generation means, the modulation information being related to the modulation process performed to generate the modulated audio signal.

### [Brief Description of Drawings]

FIG. 1 is a block diagram showing the configuration of a cognitive function improving system according to an embodiment of the present invention.
FIG. 2 is a block diagram showing a configuration of a content playback device according to the embodiment.
FIG. 3 is a block diagram showing a configuration of a content distribution device according to the present embodiment.
FIG. 4 is an explanatory diagram of one aspect of the present embodiment.
FIG. 5 is a flowchart of a content distribution process according to the embodiment.
FIG. 6 is a diagram showing an example of a score function used in the content distribution process of the present embodiment.
FIG. 7 is a flowchart of a content playback process according to the embodiment.
8A and 8B are diagrams showing example screens displayed in the content playback process of the embodiment.
9A to 9C are diagrams showing example screens displayed in the content playback process of the embodiment.
FIG. 10 is a flowchart of a content distribution process according to the first modified example.
FIG. 11 is a flowchart of a content playback process according to the first modified example.
FIG. 12 is a flowchart of a content playback process according to a second modified example.

### [Description of Embodiments]

Hereinafter, an embodiment of the present invention will be described in detail with reference to the drawings. In the drawings for explaining the embodiments, the same components are generally given the same reference numerals, and repeated explanations thereof will be omitted.

### (1) Configuration of cognitive function improvement system

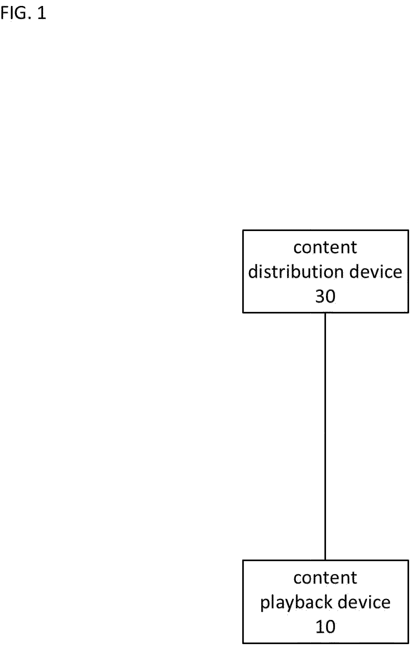

The configuration of the cognitive function improvement system will be described. FIG. 1 is a block diagram showing the configuration of a cognitive function improving system according to the present embodiment.

As shown in FIG. 1, the cognitive function improvement system 1 includes a content playback device 10 and a content distribution device 30.

A transmission path for distributing the content is provided between the content playback device 10 and the content distribution device 30. The content may be broadcast content or on-demand delivery content. In this embodiment, the content distributed includes audio content. In addition, the content distribution device 30 may distribute content including audio content and video content (for example, a TV program or an uploaded MP4 file). The transmission path may be a transmission path for information communication or a transmission path for broadcasting. The transmission path for information communication may include a network (e.g., the Internet or an intranet) or a predetermined interface (e.g., a wireless interface using Bluetooth or the like). The broadcast transmission path may include, for example, a broadcast network for TV broadcasting.

The content reproducing device 10 obtains the content distributed from the content distributing device 30 and reproduces the content. The content reproducing device 10 is an example of a signal processing device. The content playback device 10 is, for example, a TV, a set-top box, a radio, a music player, a smartphone, a PC, an electronic musical instrument, a telephone, a game machine, or an amusement machine.

The content distribution device 30 distributes content to the content playback device 10. The content distribution device 30 is an example of a signal processing device.

### (1-1) Configuration of the Content Playback Device

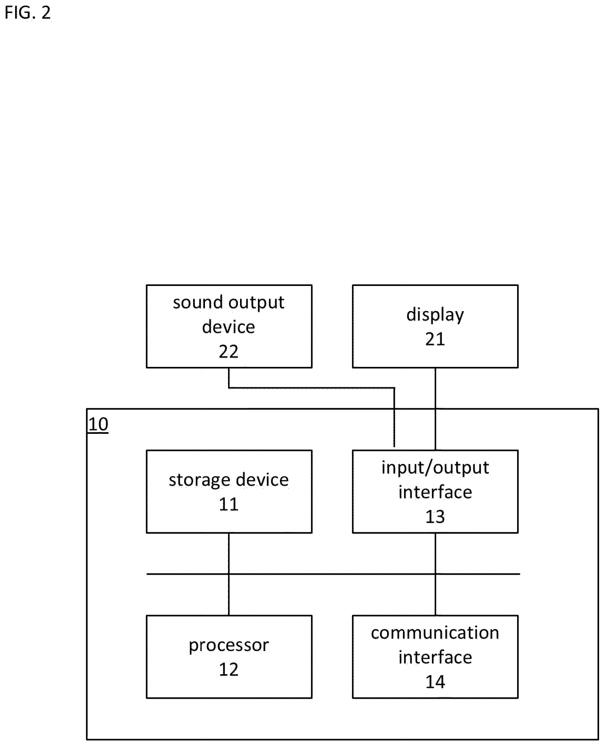

The configuration of the content reproducing device will be described. FIG. 2 is a block diagram showing the configuration of the content reproducing device of this embodiment.

As shown in FIG. 2, the content playback device 10 includes a storage device 11, a processor 12, an input/output interface 13, and a communication interface 14. The content playback device 10 is connected to a display 21 and an sound output device 22.

The storage device 11 is configured to store programs and data. The storage device 11 is, for example, a combination of a Read Only Memory (ROM), a Random Access Memory (RAM), and a storage (for example, a flash memory or a hard disk). The program and data may be provided via a network, or may be provided by being recorded on a computer-readable recording medium.

The programs include, for example, the following programs:
- OS (Operating System) programs
- Application programs that perform information processing

The data includes, for example, the following data:
- Databases referenced in information processing
- Data obtained by executing information processing (i.e., the results of executing information processing)

The processor 12 is a computer that realizes the functions of the content playback device 10 by reading and executing the programs stored in the storage device 11. At least a part of the functions of the content playback device 10 may be realized by one or more dedicated circuits. The processor 12 may be, for example, at least one of the following:
- CPU (Central Processing Unit)
- GPU (Graphic Processing Unit)
- ASIC (Application Specific Integrated Circuit)
- FPGA (Field Programmable Array)
0 DSP (Digital Signal Processor)

The input/output interface 13 is configured to obtain information (e.g., user instructions) from an input device connected to the content playback device 10, and to output information (e.g., an image signal, an audio signal, or a control signal) to an output device connected to the content playback device 10.

The input device is, for example, a physical button, a keyboard, a pointing device, a touch panel, or a combination thereof.

The output device is, for example, a display 21, an sound output device 22, or a combination thereof.

Furthermore, the input/output interface 13 may include signal processing hardware such as, for example, an A/D converter, a D/A converter, an amplifier, a mixer, a filter, and the like.

The communication interface 14 is configured to control communication between the content playback device 10 and an external device (e.g., the content distribution device 30).

The display 21 is configured to display an image (still image or video image). The display 21 is, for example, a liquid crystal display or an organic EL display.

The sound output device 22 generates a sound according to the output audio signal. The sound output device 22 may include, for example, a loudspeaker (it may include a speaker with a built-in amplifier (a powered speaker)), headphones, earphones, or smart speakers.

The sound output device 22 is connected to the processor 12 via a predetermined interface capable of transmitting an audio signal, and obtains an output audio signal from the processor 12. The interface is, for example, a Sony Philips Digital Interface (SPDIF), a High-Definition Multimedia Interface (HDMI (registered trademark)), a pin connector (RCA pin), or an audio interface for headphones. The interface may be a wireless interface using Bluetooth (registered trademark) or the like. The audio signal in this embodiment includes either an analog signal or a digital signal, or both.

### (1-2) Configuration of Content Distribution Device

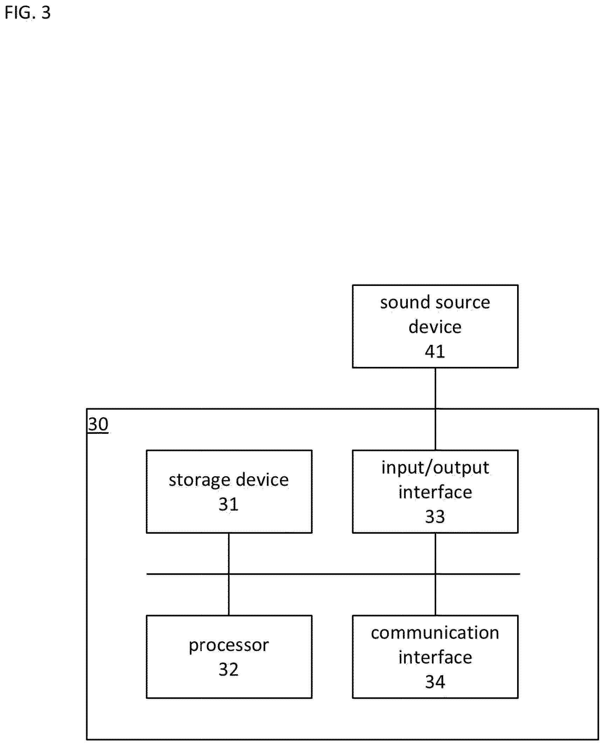

The configuration of the content distribution device will be described. FIG. 3 is a block diagram showing the configuration of the content distribution device of this embodiment.

As shown in FIG. 3, the content distribution device 30 includes a storage device 31, a processor 32, an input/output interface 33, and a communication interface. The content distribution device 30 is connected to a sound source device 41.

The storage device 31 is configured to store programs and data. The storage device 31 is, for example, a combination of ROM, RAM, and storage (for example, flash memory or a hard disk). The program and data may be provided via a network, or may be provided by being recorded on a computer-readable recording medium.

The programs include, for example, the following programs:
- OS program
- Application programs that perform information processing

The data includes, for example, the following data:
- Databases referenced in information processing
- Results of information processing

The processor 32 is a computer that realizes the functions of the content distribution device 30 by reading and executing the programs stored in the storage device 31. At least a part of the functions of the content distribution device 30 may be realized by one or more dedicated circuits. The processor 32 may be, for example, at least one of the following:
- CPU
- GPU
- ASIC
- FPGA
- DSP

The input/output interface 33 is configured to receive information (e.g., user instructions or audio signals) from an input device connected to the content distribution device 30, and to output information (e.g., image signals) to an output device connected to the content distribution device 30. The input device is, for example, a sound source device 41, a physical button, a keyboard, a pointing device, a touch panel, or a combination thereof.

The output device is, for example, a display, a speaker, or a combination thereof.

The communication interface 34 is configured to control communication between the content distribution device 30 and an external device (e.g., the content playback device 10).

The sound source device 41 is configured to generate and transmit an input audio signal.

### (2) One aspect of the embodiment

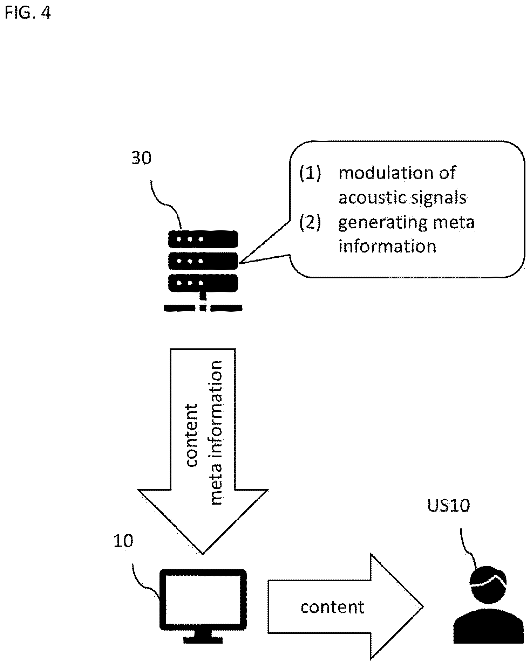

One aspect of this embodiment will be described. FIG. 4 is an explanatory diagram of one aspect of this embodiment.

As shown in FIG. 4, the content distribution device 30 performs amplitude modulation processing on the audio signal. In addition, the content distribution device 30 generates meta information regarding the content for improving cognitive function. The meta information may include, for example, information regarding the amplitude modulation performed on the audio signal (hereinafter referred to as "modulation information"). The content distribution device 30 distributes to the content playback device 10 the content including the audio signal (output audio signal) generated by the amplitude modulation process, and the meta information.

The content reproducing device 10 reproduces the content acquired from the content distribution device 30. The content reproducing device 10 causes the sound output device 22 to output an audio signal (output audio signal) included in the content. Because the output audio signal has amplitude changes corresponding to the frequency of gamma waves, when the user US10 hears the sound produced by the output audio signal, gamma waves are induced in the brain of the user US10 (brain waves are synchronized to the gamma frequency). This is expected to have the effect of improving cognitive function (e.g., treating or preventing dementia). Here, the content reproducing device 10 controls the playback of the content based on the meta information acquired from the content distribution device 30. As an example, the content playback device 10 displays information based on the meta-information (for example, information regarding the degree of improvement in cognitive function that can be expected from viewing the content) on the display 21. In the following description, the term "viewing" is used to include the meaning of "listening." By checking such information, the user US10 can easily determine whether to continue viewing the content being played or switch to other content, or for how long to continue viewing the content being played.

### (3) Information processing

The information processing of this embodiment will be described.

### (3-1) Content distribution processing

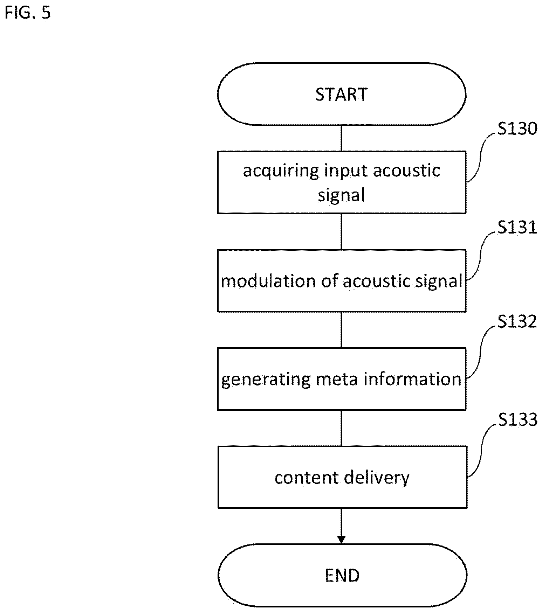

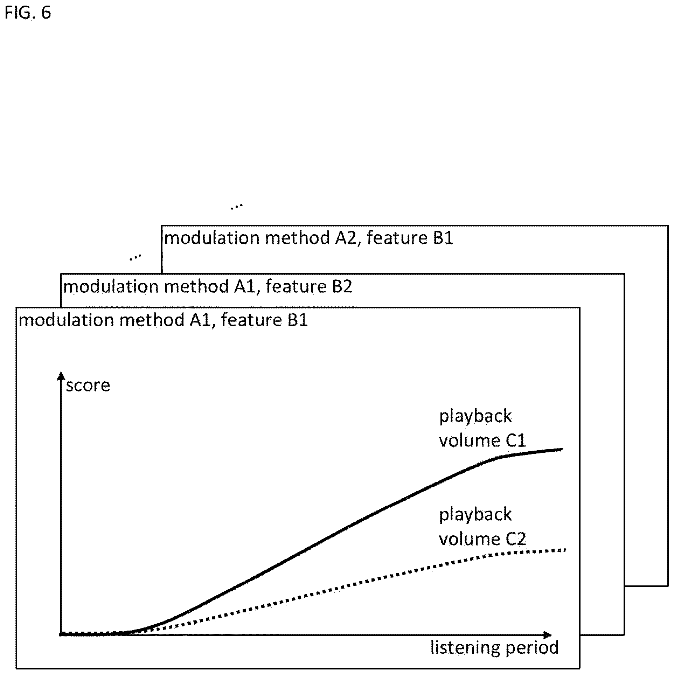

The content distribution process of this embodiment will be described. FIG. 5 is a flowchart of the content distribution process of this embodiment. FIG. 6 is a diagram showing an example of a score function used in the content distribution process of this embodiment.

The content distribution process in FIG. 5 is realized by the processor 32 of the content distribution device 30 reading and executing a program stored in the storage device 31.

The content distribution process in FIG. 5 starts when any one of the following start conditions is met.
- The content distribution process in FIG. 5 is called by another process or an external instruction.
- The user of the content playback device 10 performs an operation for calling up the content distribution process of FIG.
- The content distribution device 30 enters a predetermined state (for example, power is turned on).
- The specified date and time has arrived.
- A predetermined time has elapsed since a predetermined event (for example, the start-up of the content distribution device 30 or the previous execution of the content distribution process in FIG. 5).

As shown in FIG. 5, the content distribution device 30 acquires an input audio signal (S130).

Specifically, the content distribution device 30 receives an input audio signal sent from a sound source device 41. Alternatively, the processor 32 may read content including the input audio signal from the storage device 31 and extract (which may include demultiplexing and decoding) the input audio signal.

In step S130, the content distribution device 30 may further perform A/D conversion of the input audio signal.

The input audio signal may correspond, for example, to at least one of the following:
- Musical content (e.g., singing, playing, or a combination thereof (i.e., a song). It may include music content that accompanies the video content.)
- Audio content (e.g., readings, narrations, announcements, radio plays, solo performances, conversations, monologues, or combinations thereof. This may include audio content accompanying video content.)
- Other audio content (e.g., electronic, environmental, or mechanical sounds)

However, singing, or audio content, is not limited to sounds produced by the human vocal tract, but may also include sounds generated by voice synthesis technology.

After step S130, the content distribution device 30 performs modulation of the audio signal (S131). Specifically, the content distribution device 30 generates an output audio signal by at least partially amplitude modulating the input audio signal acquired in step S130.

As a first example of modulating an audio signal (S131), the content distribution device 30 generates an intermediate audio signal including a plurality of audio signals having different characteristics based on an input audio signal. Here, the features may be determined based on an input operation by a creator or provider of the content corresponding to the input audio signal, or on an external instruction, or may be determined by an algorithm. For example, the content distribution device 30 may determine the characteristics for generating the intermediate audio signal based on the result of analyzing the input audio signal.

The characteristics may be, for example, at least one of the following:
- Sound characteristics (especially qualitative characteristics)
- Frequency characteristics
- Time characteristics
- Amplitude characteristics
- Output characteristics

The content distribution device 30 selects one or more audio signals (hereinafter referred to as "target signals") to which amplitude modulation is to be applied from the multiple audio signals included in the intermediate audio signal. Which audio signal is selected as the target signal may be determined based on an input operation by the creator or provider of the content corresponding to the input audio signal, or an external instruction, or may be determined by an algorithm. For example, the content distribution device 30 may determine the target signal based on characteristics of the audio signal (balance between voice and music, volume change, type of music, timbre, or other characteristics). This enables the content distribution device 30 to select a target signal so that the effect of improving cognitive function through modulation is greater, or to select a target signal so that the discomfort felt by the viewer of the content (i.e., the user of the content playback device 10) is minimized. However, the content distribution device 30 may treat all of the multiple audio signals included in the intermediate audio signal as target signals.

The content distribution device 30 performs amplitude modulation on the selected target signal. As an example, the content distribution device 30 performs amplitude modulation on the target signal using a modulation function having a frequency corresponding to gamma waves (for example, a frequency not less than 35 Hz and not more than 45 Hz). Specifically, assuming that a modulation function having a periodicity of 35 Hz or more and 45 Hz or less is A(t), a function representing the waveform of the audio signal before modulation is X(t), and a function representing the waveform of the modulated audio signal is Y(t), it becomes
Y(t)=A(t) • X(t)

As a result, a change in amplitude corresponding to the frequency is added to the target signal. Here, the content distribution device 30 may determine a modulation function to be used for amplitude modulation of the target signal or a modulation depth of the amplitude modulation. The modulation function or modulation depth may be determined based on an input operation by a creator or provider of the content corresponding to the input audio signal, or an external instruction, or may be determined by an algorithm. Furthermore, the content distribution device 30 may determine a common modulation function or modulation depth for a plurality of target signals, or may determine a modulation function or modulation depth for each of a plurality of target signals.

The content distribution device 30 generates an output audio signal based on audio signals that were not selected as target signals (hereinafter referred to as "non-target signals") from among the multiple audio signals included in the intermediate audio signal, and the modulated target signal. That is, the content distribution device 30 converts the non-target signal and the modulated target signal into an output audio signal. Specifically, the content distribution device 30 combines two or more audio signals from the non-target signal and the modulated target signal, or extracts or separates an audio signal from at least one of the non-target signal and the modulated target signal. The method of synthesizing the audio signals is not limited, but may include, for example, signal summation processing, HRTF (Head Related Transfer Function) convolution processing, convolution processing of a transfer function that provides position information of the sound source, or summation processing after performing these convolution processes. Furthermore, the content distribution device 30 may further perform at least one of amplification, volume adjustment, and D/A conversion of the output audio signal. On the other hand, if the asymmetric signal and the modulated target signal match the format of a deliverable audio signal or the output format of the content playback device 10 (for example, if the asymmetric signal and the modulated target signal correspond to multi-channel audio signals associated with each speaker that constitutes a surround system as the content playback device 10), such conversion is not necessary. In this case, the non-target signal and the modulated target signal are treated as the output audio signal.

In a second example of modulating an audio signal (S131), the input audio signal includes a plurality of audio signals with different characteristics. The content distribution device 30 selects one or more audio signals to which amplitude modulation is to be applied from among a plurality of audio signals included in the input audio signal. The second example of the modulation of the audio signal (S131) can be understood by appropriately replacing the "intermediate audio signal" with the "input audio signal" in the explanation of the first example above.

In the third example of the modulation of an audio signal (S131), the content distribution device 30 performs amplitude modulation on the input audio signal. The amplitude modulation of the input audio signal is similar to the amplitude modulation of the target signal described in the first example of the modulation of the audio signal (S131). As a result, a change in amplitude corresponding to the frequency of gamma waves is added to the input audio signal. Here, the content distribution device 30 may determine a modulation function to be used for the amplitude modulation of the input audio signal or a modulation depth of the amplitude modulation. The modulation function or modulation depth may be determined based on an input operation by a creator or provider of the content corresponding to the input audio signal, or an external instruction, or may be determined by an algorithm.

The content distribution device 30 generates an output audio signal based on the modulated input audio signal. That is, the content distribution device 30 converts a modulated input audio signal into an output audio signal. Specifically, when the modulated input audio signal is made up of multiple audio signals, the content distribution device 30 combines two or more of the multiple audio signals, or extracts or separates an audio signal from the modulated input audio signal. The details of the method for synthesizing the audio signals are as explained in the first example of the modulation of the audio signal (S131). On the other hand, if the modulated input audio signal matches the format of a deliverable audio signal or the output format of the content playback device 10 (for example, if the modulated input audio signal corresponds to a multi-channel audio signal associated with each speaker that constitutes a surround system as the content playback device 10), such conversion is not necessary. In this case, the modulated input audio signal is treated as the output audio signal.

After step S131, the content distribution device 30 generates meta information (S132).

Specifically, the content distribution device 30 generates meta information according to the processing performed in modulating the audio signal (S131). The meta information may include, for example, at least one of the following pieces of information:
- Information (e.g., a flag) indicating that it is possible to select sound playback based on the output audio signal (i.e., the audio signal to which amplitude modulation has been applied)
- Modulation information
- Feature information
- Ideal viewing conditions for the content (e.g., playback volume)
- Type of content
- Score

The modulation information is information about the processing that was performed to generate the output audio signal included in the content. For example, the modulation information may include information regarding at least one of the following:
- Modulation function used for amplitude modulation
- Modulation depth
- Amplitude modulated audio signal
- Audio signal with no amplitude modulation applied

Here, when an intermediate audio signal including multiple audio signals is generated based on an input audio signal, and a decision is made individually as to whether or not to apply amplitude modulation to the multiple audio signals, each of the multiple audio signals corresponds to either an "audio signal to which amplitude modulation has been applied" or an "audio signal to which amplitude modulation has not been applied." Similarly, when the input audio signal consists of multiple audio signals and a decision is made individually as to whether or not to apply amplitude modulation to each of the multiple audio signals, each of the multiple audio signals corresponds to either an "audio signal with amplitude modulation applied" or an "audio signal without amplitude modulation applied." In these cases, since the function used for amplitude modulation or the modulation depth may be different for each of the audio signals to which amplitude modulation is applied, the modulation information may include information regarding the "function used for amplitude modulation" or "modulation depth" of each audio signal.

The feature information is information on the feature of an audio signal to which amplitude modulation has been applied in order to generate an output audio signal included in the content. For example, the feature information may include information regarding at least one of the following:
- Amplitude, energy or rms distribution in the frequency domain (e.g. the result of a Fast Fourier Transform (FFT))
- Distribution of amplitude, energy or effective value in the time domain
- Sound source type (e.g., vocal, instrument, object, music, dialogue, natural sound, electronic sound, etc.)
- Sound source type for each playback section
- Content attributes (e.g., program information for a TV program in which the audio signal is included)

The score is information regarding the magnitude of the effect of improving cognitive function that is expected when the user listens to the sounds contained in the played back content under an ideal listening environment. Regarding the calculation of the score, as an example, the content distribution device 30 can reference a score function to derive a score corresponding to some or all of the following elements:
- The length of time the content (output audio signal) will continue to be played (i.e., listening duration)
- Content playback volume in an ideal listening environment

Modulation method of amplitude modulation applied to an audio signal
- Features of audio signals to which amplitude modulation has been applied The score function may be saved in the storage device 31, or may be stored in a storage device provided in an external device (e.g., a server) accessible by the content distribution device 30.

Here, the score represents the degree of improvement in cognitive function, and is defined to correlate with the estimated results of the amount of EEG elicitation by sound stimulation (i.e., the extent to which gamma waves were induced), an index calculated from the degree of EEG synchronization at each time (i.e., the duration for which gamma wave synchronization was achieved), or an index calculated from the EEG elicitation ratio at each time (i.e., the history of the extent to which gamma waves were induced compared to other EEG bands). As an example, the score is determined to increase (e.g., increase linearly) with increasing amount of electroencephalography evoked. As another example, the score is determined so that it increases very little when the amount of brainwave evoked is below the first threshold, and increases in response to an increase in the amount of brainwave evoked after the amount of brainwave evoked exceeds the first threshold. As yet another example, the score is determined so that it increases in response to an increase in the amount of brainwave evoked energy when the amount of brainwave evoked energy is below the second threshold, and then increases very little (i.e., saturates) once the amount of brainwave evoked energy exceeds the second threshold. By having a subject listen to various audio signals under various conditions and measuring the brain wave elicitation of the subject with an electroencephalograph, it is possible to describe the relationship between the elements contained in the meta information and the brain wave elicitation. A score function for deriving a score corresponding to an element included in the meta information can be created based on the relationship between the element included in the meta information and the amount of brain wave elicitation, and the relationship between the score and the amount of brain wave elicitation. The score function may be defined as a mathematical expression that uses the above elements as arguments, or may be defined as a lookup table.

As an example, the content distribution device 30 calculates the score using the score function shown in FIG. In the score function of FIG. 6, the score is defined to increase or maintain (in other words, not decrease) with increasing listening period. Moreover, the score function in FIG. 6 is defined so that the score for a playback volume C1 is higher than the score for a playback volume C2 (lower than C1).

The content distribution device 30 may determine details of the modulation process to be performed in step S131 so that the score or the increment of the score is equal to or greater than a reference value. As an example, the content distribution device 30 may change the modulation settings and re-execute step S131 if the score calculated in step S132 falls below a reference value or if the cumulative score value estimated from the current score increment falls below a reference value. The content distribution device 30 may perform modulation of the audio signal (S131) after generating the meta information (S132). In this case, for example, the content distribution device 30 may generate meta information indicating a predetermined score, and then determine the details of the modulation process so that the score of the modulated content will be equal to or higher than that score, and perform the modulation process on the content.

After step S132, the content distribution device 30 executes content distribution (S133).

Specifically, the content distribution device 30 distributes the content including the output audio signal generated in step S131 to the content playback device 10 together with the meta information generated in step S132. As an example, the content distribution device 30 may generate a content file in a predetermined format by multiplexing an output audio signal and meta information. Alternatively, the content distribution device 30 may distribute the content file and the meta information through separate transmission paths. For example, the content distribution device 30 may distribute the content file by normal broadcasting, and distribute the meta information by data broadcasting or over the Internet.

The content distribution device 30 may also include the raw audio signal in the content file. The raw audio signal may be identical to the output audio signal except for the presence or absence of amplitude modulation. A viewer of the content can choose whether to hear sound based on the raw audio signal or sound based on the output audio signal. For example, the content distribution device 30 may distribute an unprocessed audio signal as main audio data in a TV program, and distribute an output audio signal as secondary audio data. For example, the content distribution device 30 may distribute an unprocessed audio signal as audio data for a first subchannel of a multi-channel TV program, and distribute an output audio signal as audio data for a second subchannel. In this case, a TV viewer can easily select whether to play back the raw audio signal or the output audio signal by switching between the main audio and the secondary audio or by switching the viewing channel. Furthermore, the content distribution device 30 may include an image signal (a moving image signal or a still image signal) in the content file. Furthermore, the content distribution device 30 may distribute a plurality of contents including the same image signal and different audio signals (for example, an output audio signal and an unprocessed audio signal). Furthermore, the content distribution device 30 may perform encoding (compression) processing on various types of information before multiplexing it.

After step S133, the content distribution device 30 ends the content distribution process of FIG.

The content distribution device 30 may perform the content distribution process of FIG. 5 all at once for an input audio signal having a fixed playback period (e.g., one piece of music content), or may repeatedly perform the content distribution process of FIG. 5 for each predetermined playback section of the input audio signal (e.g., every 100 ms). Alternatively, the content distribution device 30 may continuously perform modulation processing on the input audio signal, such as modulation by analog signal processing, and output the modulated audio signal. The content distribution process shown in FIG. 5 may be terminated in response to a specific termination condition (e.g., the passage of a certain amount of time, an operation being performed by the creator or provider of the content or a user of the content playback device 10, or the output history of the modulated sound reaching a predetermined state).

### (3-2) Content playback process

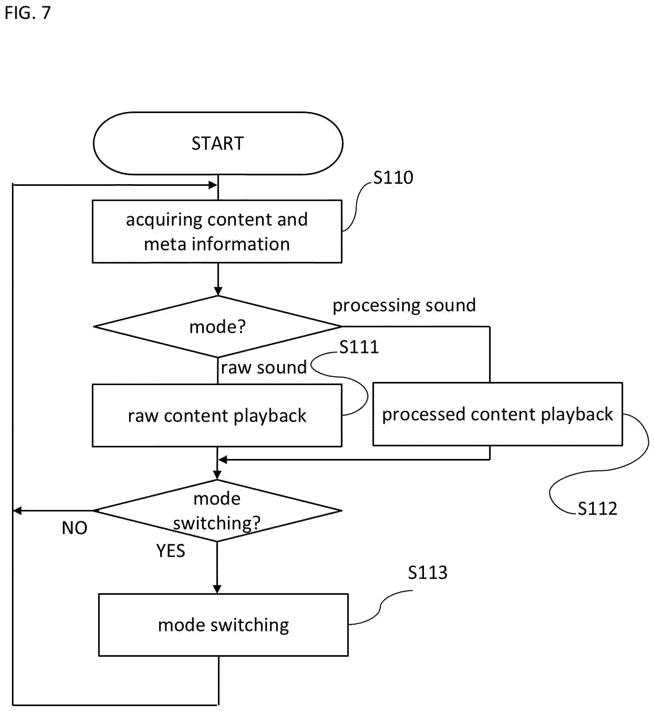

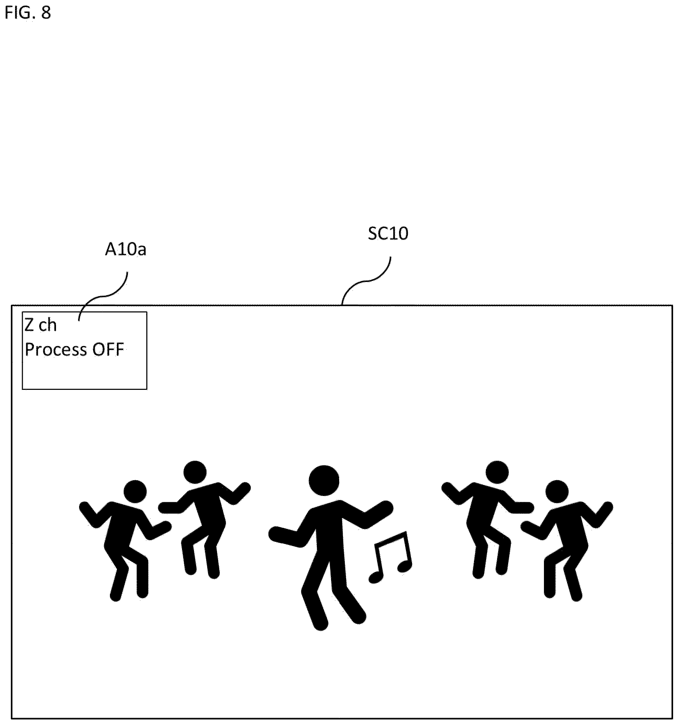

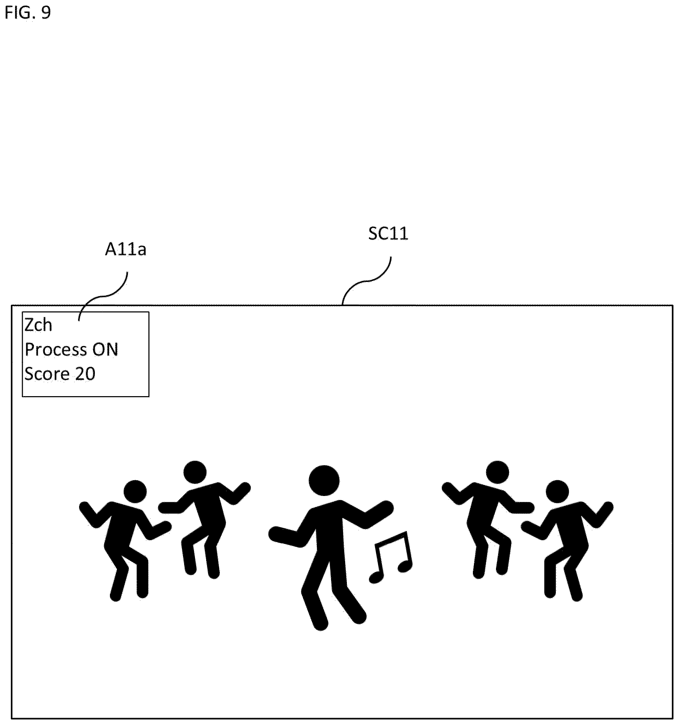

The content playback process of this embodiment will be described. FIG. 7 is a flowchart of the content playback process of this embodiment. FIG. 8 is a diagram showing an example of a screen displayed in the content playback process of this embodiment. FIG. 9 is a diagram showing an example of a screen displayed in the content playback process of this embodiment.

The content playback process in FIG. 7 starts when any one of the following start conditions is met:
- The content playback process in FIG. 7 is called by another process or an external instruction.
- The user performs an operation to call up the content playback process in FIG. 7.
- The content playback device 10 enters a predetermined state (for example, power is turned on).
- The specified date and time has arrived.
- A predetermined time has elapsed since a predetermined event (for example, the start-up of the content playback device 10 or the previous execution of the content playback process in FIG. 7).

As shown in FIG. 7, the content playback device 10 acquires the content and meta information (S110). Specifically, the content playback device 10 acquires the content and meta information distributed by the content distribution device 30.

In the following description, content is taken to include raw audio signals, output audio signals, and video or still image signals. The content reproducing device 10 is capable of selecting either an unprocessed audio signal or an output audio signal, and reproducing sound based on the selected audio signal. In addition, the audio mode described later refers to, for example, the method of playing audio by the content playback device 10, and in the following explanation, the mode in which sound based on an unprocessed audio signal is referred to as the "unprocessed sound mode," and the mode in which sound based on an output audio signal is referred to as the "processed sound mode." However, the audio mode is not limited to this example and can be defined arbitrarily.

After step S110, if the raw sound mode is selected, the content reproducing device 10 reproduces the raw content (S111).

Specifically, the content reproducing device 10 reproduces the unprocessed content among the contents acquired in step S110. Here, the content playback device 10 may perform demultiplexing of the content (file), or decoding of the encoded data, or a combination of these.

As an example, the content reproducing device 10 outputs sound based on an unprocessed audio signal from the sound output device 22 and displays a moving image or a still image on the display 21. This allows the user to view a moving image or still image synchronized with a sound based on an unprocessed audio signal while listening to the sound. As an example, the content playback device 10 displays a screen SC10 of FIG.

The screen SC10 includes an object A10a.

The object A 10a displays information about the content being played. As an example, object A10a displays at least one of the following:
- Information to identify the content being played (e.g., content name, channel name)
- Information indicating that the audio mode is raw audio mode
- User score status

Object A10a may be displayed at all times, or may be displayed in response to a predetermined trigger (e.g., a channel change, an audio mode change, or a change between multiple pieces of content being played in succession) and then only for a predetermined period of time. It should be noted that the method of presenting information about the content being played back is not limited to the example shown in FIG. For example, the content playback device 10 may present information about the content being played back to the user by a voice announcement or by illuminating an LED.

When displaying the user's score status, the content playback device 10 records the user's content viewing history and calculates the user's score status at any given time by referring to the record. As an example, the content playback device 10 may calculate the transition of the cumulative score during the target period (i.e., the change in cumulative score over time) by accumulating the scores corresponding to each of a plurality of viewing histories. Alternatively, in a case where a target score for the user in a target period is set, the content playback device 10 may calculate the ratio of the cumulative score to the target score as the user's degree of achievement or non-achievement. The target score corresponds to the standard score (i.e., the sounds that should be listened to) that the user should achieve within the target period in order to improve cognitive function. The content playback device 10 may calculate the degree of achievement at a single point in time, or may calculate the change in the degree of achievement over a target period (that is, the change in the degree of achievement over time).

After step S110, if the processed sound mode is selected, the content reproducing device 10 reproduces the processed content (S112).

Specifically, the content reproducing device 10 reproduces the processed content among the contents acquired in step S110. Here, the content playback device 10 may perform demultiplexing of the content (file) (dividing the multiplexed content), or decoding of the encoded data, or a combination of these.

As an example, the content reproducing device 10 outputs a sound based on the output audio signal from the sound output device 22 and displays a moving image or a still image on the display 21. This allows the user to view a moving image or still image synchronized with a sound based on the output audio signal while listening to the sound. As an example, the content playback device 10 displays a screen SC11 of FIG. 9.

The screen SC11 includes an object A11a.

The object A11a displays information about the content being played back. As an example, object A11a displays at least one of the following:
- Information to identify the content being played (e.g., content name, channel name)
- Information indicating that the audio mode is processed sound mode
- Meta information of the content (e.g., the score corresponding to the content)
- User score status

The object A11a may be displayed at all times, or may be displayed in response to a predetermined trigger (e.g., a channel change, an audio mode change, or a change between multiple pieces of content being played in succession) and then only for a predetermined period of time. Furthermore, the meta information of the content may be presented as information separate from the content, such as a program guide (including an EPG (Electronic Program Guide)) or data broadcasting.

When the content reproducing device 10 detects a switch to the processed sound mode during playback of the unprocessed content (S111), the content reproducing device 10 executes a mode switch (S113).

For example, when the input device receives an instruction from a user to switch to the processed sound mode, the content playback device 10 executes switching to the processed sound mode.

Similarly, when the content reproducing device 10 detects a switch to the unprocessed sound mode during playback of processed content (S112), it executes mode switching (S113).

For example, when the input device receives an instruction from the user to switch to the unprocessed sound mode, the content playback device 10 executes switching to the unprocessed sound mode.

The content playback process in FIG. 7 ends when, for example, any one of the following ending conditions is met:
- The content being played has been changed.
- The content being played has ended.
- The content playback process in FIG. 7 is ended by another process or an external instruction.
- The user performs an operation to end the content playback process in FIG.
- The content playback device 10 enters a predetermined state (for example, power loss).
- The specified date and time has arrived.
- A predetermined time has elapsed since a predetermined event (for example, the start of the content
playback device 10 or the start of execution of the content playback process in FIG. 7).

### (4) Summary

As described above, the content distribution device 30 of this embodiment performs modulation processing including amplitude modulation on an audio signal to generate an audio signal (i.e., an output audio signal) having an amplitude change corresponding to the frequency of gamma waves, and outputs content including the audio signal together with corresponding meta information. By referring to the output meta information, the user of the content playback device 10 can easily determine the content to be played back or the viewing mode of the content.

The content distribution device 30 may perform modulation processing on an audio signal included in the content so that the score corresponding to the content is equal to or greater than a reference value. This allows the content playback device 10 to obtain a score equal to or greater than the reference value by viewing the content without the user being particularly conscious of the content to be played back.

The content playback device 10 of this embodiment acquires meta information corresponding to content including an audio signal (i.e., an output audio signal) having an amplitude change corresponding to the frequency of gamma waves, and controls the playback of the content based on the meta information. This allows a user to understand the meta information of the content and view the processed content without any special knowledge. As another example of the above control, the content playback device 10 may display information based on meta information corresponding to the content on a screen that displays an image included in the content. This allows the user to easily understand the meta information of the content.

The meta information may include at least one of information indicating whether or not modulation processing is applied, information indicating details of the modulation processing, and information indicating a score determined based on the details of the modulation processing. This makes it possible to perform appropriate playback processing on the content and to understand the cognitive function improvement effects that users can expect from viewing processed content.

The output audio signal may have amplitude variations corresponding to frequencies between 35 Hz and 45 Hz. This can induce gamma waves in the brain of the user of the content playback device 10, thereby improving cognitive function.

### (5) Modifications

A modification of this embodiment will now be described.

### (5-1) Modification 1

Modification example 1 will be described. The first modification is an example in which the content reproducing device modulates an audio signal.

### (5-1-1) Information processing

The information processing of the first modification will be described.

### (5-1-1-1) Content distribution processing

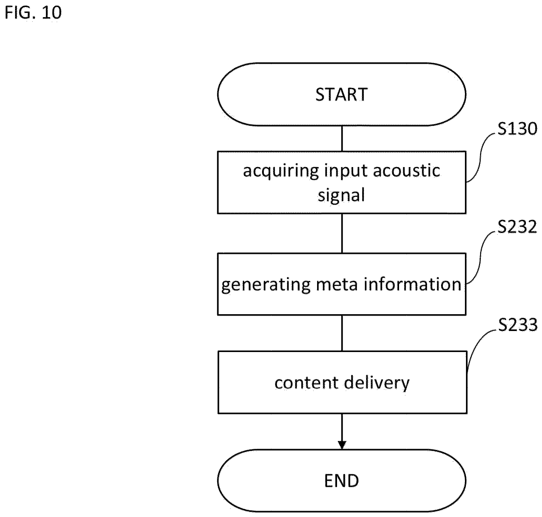

The content distribution process of the first modification will be described. FIG. 10 is a flowchart of a content distribution process according to the first modification.

The content distribution process of FIG. 10 is realized by the processor 32 of the content distribution device 30 reading and executing a program stored in the storage device 31.

The content distribution process in FIG. 10 can be started in response to the establishment of the same start condition as the content distribution process of the present embodiment.

As shown in FIG. 10, the content distribution device 30 acquires an input audio signal (S130) in the same manner as in FIG. 6.

After step S131, the content distribution device 30 generates meta information (S232).

Specifically, the content distribution device 30 generates meta information according to the modulation process that is recommended to be performed by the content playback device 10. The meta information may include the information described in this embodiment. The modulation processing recommended to be performed in the content playback device 10 may be determined based on an input operation by the creator or provider of the content corresponding to the input audio signal, or an external instruction, or may be determined by an algorithm. However, in order to calculate the score, the content distribution device 30 may perform modulation processing based on modulation information included in the meta information.

After step S232, the content distribution device 30 executes content distribution (S233).

Specifically, the content distribution device 30 distributes the content based on the input audio signal acquired in step S130 (which does not have to include processed content) to the content playback device 10 together with the meta information generated in step S232. As an example, the content distribution device 30 may generate a content file in a predetermined format by multiplexing an unprocessed audio signal (e.g., an input audio signal) and meta information. A viewer of the content can select whether to listen to a sound based on an unprocessed audio signal or a sound based on an audio signal that has been amplitude modulated by the content reproducing device 10. Furthermore, the content distribution device 30 may include an image signal (a moving image signal or a still image signal) in the content file. Furthermore, the content distribution device 30 may perform encoding (compression) processing on various types of information before multiplexing it.

After step S233, the content distribution device 30 ends the content distribution process of FIG. The content distribution device 30 may perform the content distribution process of FIG. 10 all at once for an input audio signal having a fixed playback period (e.g., one piece of music content), or may repeatedly perform the content distribution process of FIG. 10 for each specified playback section of the input audio signal (e.g., every 100 ms). Alternatively, the content distribution device 30 may continuously perform modulation processing on the input audio signal, such as modulation by analog signal processing, and output the modulated audio signal. The content distribution process shown in FIG. 10 may be terminated in response to a specific termination condition (e.g., the passage of a certain amount of time, an operation being performed by the creator or provider of the content or a user of the content playback device 10, or the output history of the modulated sound reaching a predetermined state).

### (5-1-1-2) Content playback process

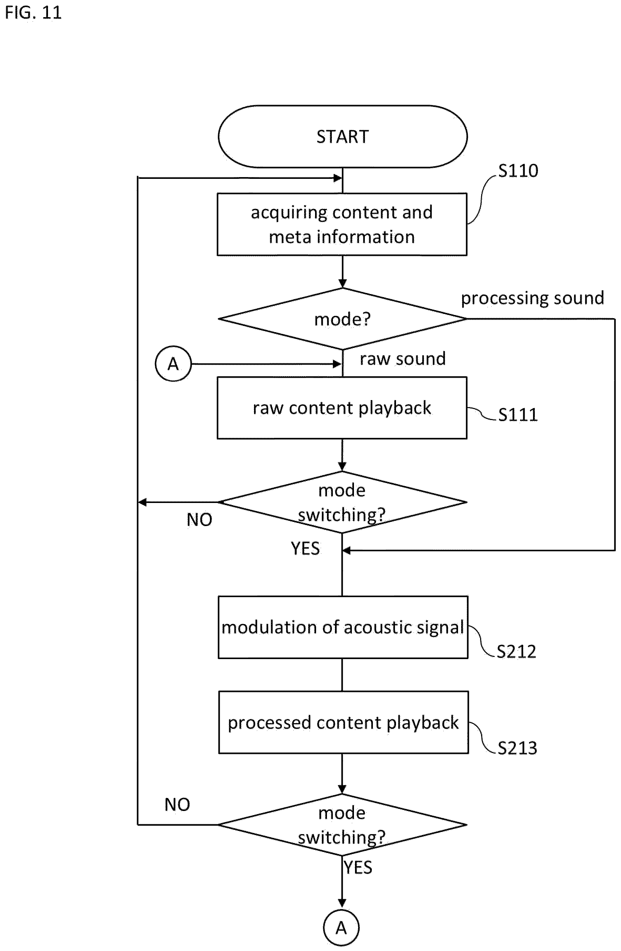

The content playback process of the first modification will be described. FIG. 11 is a flowchart of the content playback process according to the first modification.

The content playback process in FIG. 11 can be started in response to the establishment of the same start condition as in the content playback process of this embodiment (FIG. 7).

As shown in FIG. 11, the content reproducing device 10 acquires the content and meta information (S110) in the same manner as in FIG.

In the following description, content is taken to include raw audio signals and video or still image signals. The content reproducing device 10 is capable of selecting either an unprocessed audio signal or an audio signal (output audio signal) obtained by amplitude-modulating at least a portion of the unprocessed audio signal, and reproducing sound based on the selected audio signal.

After step S110, if the raw sound mode has been selected, the content reproducing device 10 reproduces the raw content (S111) in the same manner as in FIG. 7

After step S110, if the processed sound mode is selected, the content reproducing device 10 executes modulation of the audio signal (S212).

Specifically, the content playback device 10 generates an output audio signal by at least partially amplitude modulating the raw audio signal included in the content acquired in step S110. Here, the content playback device 10 may perform demultiplexing of the content (file), or decoding of the encoded data, or a combination of these.

As a first example of modulating the audio signal (S212), the content reproducing device 10 generates an intermediate audio signal based on the raw audio signal (input audio signal). The content playback device 10 may determine the method of generating the intermediate audio signal by referring to the meta information acquired in step S110.

The content playback device 10 selects a target signal from a plurality of audio signals included in the intermediate audio signal. The content playback device 10 may refer to the meta information to determine which of the audio signals to select as the target signal.

The content reproducing device 10 performs amplitude modulation on the selected target signal. The content playback device 10 may determine at least one of the modulation function and the modulation depth by referring to the meta information.

The content playback device 10 generates an output audio signal based on the non-target signal and the modulated target signal. The technique for generating an output audio signal based on the non-target signal and the modulated target signal may be similar to this embodiment.

In a second example of modulating the audio signal (S212), the raw audio signal includes multiple audio signals with different characteristics. The content reproducing device 10 selects one or more audio signals to which amplitude modulation is to be applied from among a plurality of audio signals included in the raw audio signal. The second example of the modulation of the audio signal (S212) can be understood by appropriately replacing the "intermediate audio signal" with the "unprocessed audio signal" in the explanation of the first example above.

In a third example of the modulation of the audio signal (S212), the content reproducing device 10 performs amplitude modulation on the raw audio signal. The amplitude modulation of the raw audio signal is similar to the amplitude modulation of the target signal described in the first example of the modulation of the audio signal (S212).

The content playback device 10 generates an output audio signal based on the modulated audio signal. The technique for generating an output audio signal based on the modulated audio signal may be similar to that of the present embodiment.

After step S212, the content reproducing device 10 reproduces the processed content (S213). Specifically, the content reproducing device 10 reproduces the output audio signal (processed content) generated in step S212.

As an example, the content reproducing device 10 outputs a sound based on the output audio signal from the sound output device 22 and displays a moving image or a still image on the display 21. This allows the user to view a moving image or still image synchronized with a sound based on the output audio signal while listening to the sound. As an example, the content playback device 10 displays a screen SC11 of FIG. 9.

When the content reproducing device 10 detects a switch to the processed sound mode during playback of the unprocessed content (S111), it executes modulation of the audio signal (S212).

For example, when the input device receives an instruction from a user to switch to the processed sound mode, the content playback device 10 detects the switch to the processed sound mode.

Similarly, when the content reproducing device 10 detects a switch to the unprocessed sound mode during modulation of the audio signal (S212) or playback of the processed content (S213), it executes playback of the unprocessed content (S111).

For example, when the input device receives an instruction from the user to switch to the unprocessed sound mode, the content playback device 10 detects the switch to the unprocessed sound mode.

The content playback process in FIG. 11 can be ended when the same ending condition as in the content playback process of the present embodiment is met.

### (5-1-2) Summary

As described above, the content playback device 10 of the first modified example performs modulation processing on an audio signal included in the content distributed from the content distribution device 30, based on modulation information included in the meta information. This allows the user of the content playback device 10 to view the processed content even if the content distribution device 30 does not distribute the processed content. Therefore, variant 1 is useful, for example, when the capacity of the transmission path for distributing content is limited.

In the above description of the first modification, an example has been shown in which the content playback device 10 dynamically switches modulation processing during playback of the content. However, the present invention is not limited to this, and the content reproducing device 10 may perform modulation processing on a content-by-content basis based on meta information. For example, the content playback device 10 may determine details of the modulation process to be performed in step S212 so that the score of the modulated content is equal to or greater than a reference value.

### (5-2) Modification 2

The second modification will be described. The second modification is an example in which the content playback device 10 controls the content recommended to the user and the mode in which the content is played back.

### (5-2-1) Content playback process

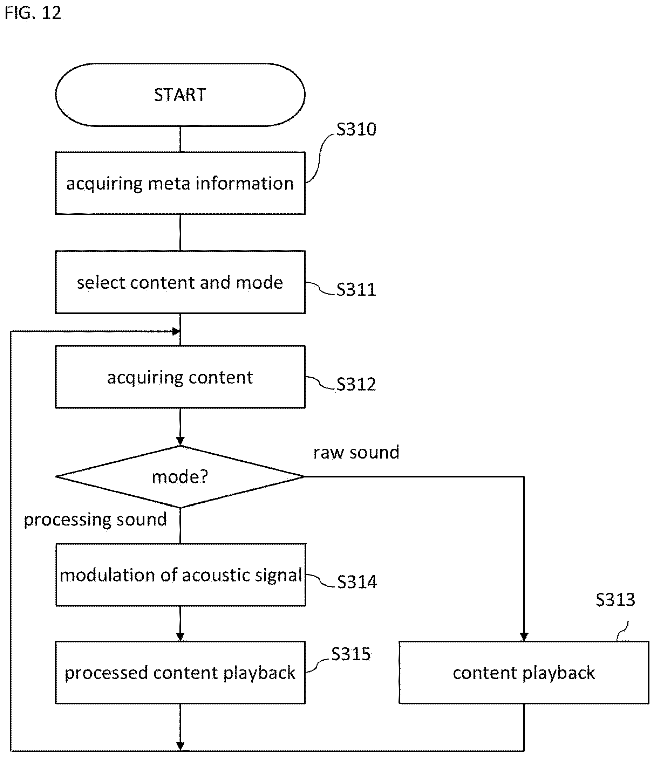

The content playback process of the second modification will be described. FIG. 12 is a flowchart of a content playback process according to the second modification.

The content playback process in FIG. 12 can be started in response to the establishment of the same start condition as in the content playback process (FIG. 7) of this embodiment.

As shown in FIG. 12, the content playback device 10 acquires meta information (S310).

Specifically, the content playback device 10 acquires meta information of a plurality of playable contents. As a first example of acquiring meta information (S310), the content playback device 10 acquires meta information distributed by the content distribution device 30. As a second example of acquiring meta information (S310), the content playback device 10 acquires the meta information by referring to an information source (for example, an EPG) in which the meta information of the content is stored. As a third example of obtaining meta information (S310), the content playback device 10 obtains meta information by demultiplexing the content stored in the storage device 11. As a fourth example of acquiring meta information (S310), the content playback device 10 acquires meta information input by the user.

After step S310, the content playback device 10 selects the content and the mode (S311).

Specifically, the content playback device 10 selects the content to be recommended to the user and the mode in which the content is to be played back, based on the meta-information acquired in step S310. As an example, the content playback device 10 may select the content or mode based on at least one of the following user information:
- User attributes (e.g., age, gender, place of origin, location, language used)
- User operation history (e.g., browsing history of sites for elderly people, operation history that may indicate dementia)
- User's content viewing history (for example, whether the user viewed enough content including sounds based on the output audio signal on the day)
- User survey response results
- User dementia test results

The content playback device 10 may, for example, estimate the user's preferences (e.g., genre, artist, etc.) based on such user information, and may select content or a mode according to the preferences. For example, the content playback device 10 may preferentially select content that is preferred by the user, or may reduce the frequency with which the content that is preferred by the user is played back in the processed sound mode.

Furthermore, the content playback device 10 may select content and a mode based on the score of each content so that the user can achieve the goal of obtaining a score. For example, the content playback device 10 may preferentially select content with a high score or content with a score exceeding a predetermined threshold.

In step S311, the content playback device 10 may select only one piece of content to be viewed by the user immediately and the mode in which that content will be played back, or it may select a combination of multiple pieces of content to be viewed by the user in the future (i.e., a playlist) and the mode in which that content will be played back. For example, the content playback device 10 may create a playlist such that the total score of the content included in the playlist is equal to or greater than a reference value. The reference value may be determined for each user, or may be common to a plurality of users. The content playback device 10 may obtain the reference value from an external device, may read it from the storage device 11, or may determine it based on user input. The selection of the content and mode in S311 may be performed automatically by the content playback device 10 based on the meta information, or may be performed in response to a user operation on a candidate proposed by the content playback device 10 based on the meta information.

After step S311, the content playback device 10 acquires the content (S312).

Specifically, the content playback device 10 acquires the content selected in step S311. As a first example of acquiring the content (S312), the content playback device 10 acquires the content distributed by the content distribution device 30. As a second example of acquiring the content (S312), the content playback device 10 acquires the content stored in the storage device 11.

After step S312, if the raw sound mode is selected, the content reproducing device 10 reproduces the raw content (S313).

Specifically, the content reproducing device 10 reproduces the unprocessed content from among the contents acquired in step S312. Here, the content playback device 10 may perform demultiplexing of the content (file), or decoding of the encoded data, or a combination of these.

As an example, the content reproducing device 10 outputs sound based on an unprocessed audio signal from the sound output device 22 and displays a moving image or a still image on the display 21. This allows the user to listen to sound based on an unprocessed audio signal while viewing a moving image or still image synchronized with the sound. As an example, the content playback device 10 displays a screen SC10 of FIG. 8.

After step S312, if the processed sound mode is selected, the content reproducing device 10 executes modulation of the audio signal (S314).

Specifically, the content playback device 10 generates an output audio signal by at least partially amplitude modulating the raw audio signal included in the content acquired in step S312. Here, the content playback device 10 may perform demultiplexing of the content (file), or decoding of the encoded data, or a combination of these. However, when the content includes an output audio signal, the content playback device 10 can omit modulating the audio signal (S314).

As a first example of modulating the audio signal (S314), the content playback device 10 generates an intermediate audio signal based on the raw audio signal (input audio signal). The content playback device 10 may determine the method of generating the intermediate audio signal by referring to the meta information acquired in step S310 (the meta information corresponding to the content acquired in step S312).

The content playback device 10 selects a target signal from a plurality of audio signals included in the intermediate audio signal. The content playback device 10 may refer to the meta information to determine which of the audio signals to select as the target signal.

The content reproducing device 10 performs amplitude modulation on the selected target signal. The content playback device 10 may determine at least one of the modulation function and the modulation depth by referring to the meta information.

The content playback device 10 generates an output audio signal based on the non-target signal and the modulated target signal. The technique for generating an output audio signal based on the non-target signal and the modulated target signal may be similar to this embodiment.

In a second example of modulating the audio signal (S314), the raw audio signal includes multiple audio signals with different characteristics. The content reproducing device 10 selects one or more audio signals to which amplitude modulation is to be applied from among a plurality of audio signals included in the raw audio signal. The second example of the modulation of the audio signal (S314) can be understood by appropriately replacing the "intermediate audio signal" with the "raw audio signal" in the explanation of the first example above.

In a third example of the modulation of the audio signal (S314), the content reproducing device 10 performs amplitude modulation on the raw audio signal. The amplitude modulation of the raw audio signal is similar to the amplitude modulation of the target signal described in the first example of the modulation of the audio signal (S314).

The content playback device 10 generates an output audio signal based on the modulated audio signal. The technique for generating an output audio signal based on the modulated audio signal may be similar to that of the present embodiment.

After step S314, the content reproducing device 10 reproduces the processed content (S315).

Specifically, the content reproducing device 10 reproduces the output audio signal (processed content) generated in step S314. However, if the content acquired in step S312 includes an output audio signal, the content playback device 10 can reproduce the output audio signal included in the content.

As an example, the content reproducing device 10 outputs a sound based on the output audio signal from the sound output device 22 and displays a moving image or a still image on the display 21. This allows the user to view a moving image or still image synchronized with a sound based on the output audio signal while listening to the sound. As an example, the content playback device 10 displays a screen SC11 of FIG.

The content playback device 10 repeats the processes of steps S312 to S313 or steps S312 and S314 to S315 until playback of the content selected in step S311 is completed. When playback of the content selected in step S311 has been completed, the content playback device 10 may re-acquire the meta information (S310) or re-select the content and mode (S311), or may terminate the content playback process of FIG. 12.

The content playback process in FIG. 12 can be ended when the same ending condition as in the content playback process of the present embodiment is satisfied.

### (5-2-2) Summary

As described above, the content playback device 10 of the second modification selects content to be recommended to a user based on the meta information of the content and the user information. This allows the user to view content that is appropriate from the standpoint of preference and the effect of improving cognitive function, without having to actively select the content to be played back.

Furthermore, the content playback device 10 may generate a list (playlist) of one or more pieces of content to be played back such that the total score of the content included in the list is equal to or greater than a reference value. This allows the user to view a group of contents that can earn a score equal to or higher than a reference value without having to actively select the content to be played back.

### (7) Other Modifications

The storage device 11 may be connected to the content playback device 10 via a network. The display 21 may be integrated with the content playback device 10. The storage device 31 may be connected to the content distribution device 30 via a network.

Each step of the above information processing can be executed by either the content playback device 10 or the content distribution device 30. Some of the steps of the above information processing may be executed by an external device (e.g., a server) not shown.

In the above description, an example in which the content playback device 10 is connected to one sound output device 22 has been shown. The content playback device 10 may be connected to a plurality of sound output devices 22, and in this case, the content playback device 10 may be configured to be able to select to which sound output device 22 the audio signal is to be sent.

The content playback device 10 may be equipped with a function for conducting a questionnaire or a cognitive function test (for example, a function for measuring cognitive function through voice dialogue with the user).

The algorithm for determining how to generate an output audio signal (e.g., the modulation method, the combining ratio of two or more audio signals, the method for determining the audio signal to which amplitude modulation is applied, etc.), or the default method for generating an output audio signal, may be changed by updating the firmware of the content distribution device 30. The firmware is updated, for example, by the content distribution device 30 communicating with an external device (for example, a server not shown).

Although an example has been given in which the input audio signal is acquired from the sound source device 41, the input audio signal may be generated by receiving environmental sound by a microphone. The input audio signal may be read from the storage device 31 or a removable medium connected to the content distribution device 30. The sound source device 41 (including the sound source device 41 built into the content distribution device 30) may generate the input audio signal each time.

In the above description, an example has been given in which an audio signal derived from an input audio signal is modulated. However, the output audio signal may also be generated based on an input audio signal and a modulated audio signal that is not derived from the input audio signal.

The above description has focused on an example in which the modulation function has a frequency of 35 Hz or more and 45 Hz or less. However, the modulation function used by the content playback device 10 or the content distribution device 30 is not limited to this, and may be any modulation function that has an effect on the induction of gamma waves in the listener's brain. For example, the modulation function may have a frequency between 25 Hz and 140 Hz. Also for example, the frequency of the modulation function may vary over time, with portions of the modulation function having frequencies below 35 Hz or above 45 Hz.

In the above description, an example has been given in which content is distributed together with meta information. However, instead of distributing the content together with the meta information, these may be recorded on a storage medium. In this case, the content distribution device can be read as a content recording device. The content playback device can read the content and meta information from the storage medium, play back the content, and perform control based on the meta information.

In the above description, an example in which the content distribution device 30 generates meta information has been mainly described, but the present invention is not limited to this. For example, the meta information may be set by a content creator, a broadcasting company, or a viewer of the content. The set meta information may be stored in a storage device or a recording medium, and the content distribution device 30 may obtain the meta information from an external storage device or a recording medium. In this case, the content distribution device 30 may acquire the content and the meta information separately, or may acquire the content to which the meta information has been added. Also, for example, the content distribution device 30 may determine the meta information based on an input by a user.

Furthermore, the meta information does not need to be distributed to the content playback device 10. For example, the meta information may be published on a web page, in a newspaper, in a magazine, or the like, or may be notified to a terminal used by a user (for example, a notification in an app or a notification by email). In this case, the content playback device 10 may acquire the meta information by, for example, referring to an information source in which the meta information of the content is stored, or may acquire the meta information input by the user.

Furthermore, when the content distribution device 30 acquires additional information (for example, an ID3 tag in an MP3 file) together with the input audio signal, the content distribution device 30 may modify the additional information and output it to an external device together with the output audio signal.

In the present embodiment, an example has been described in which the content distribution device 30 distributes content including an unprocessed audio signal and an output audio signal. Here, the number of multiplexed audio signals of the content to be distributed may be determined arbitrarily. In other words, output audio signals to which different modulation processes (e.g., modulation processes having at least one of a modulation function or a modulation depth different from each other) have been applied may be multiplexed and distributed. Furthermore, the content distribution device 30 may multiplex and distribute other audio signals in addition to the output audio signal and the unprocessed audio signal. For example, the content distribution device 30 may multiplex and distribute an unprocessed sound in a first language, a processed sound in the first language, an unprocessed sound in a second language, and a processed sound in the second language.

An amplitude-modulated audio signal (for example, an audio signal equivalent to a beep, chirp, noise, or background music) may be synthesized in a period corresponding to a silent portion of the raw audio signal. This can improve the cognitive function of the user of the content playback device 10 without interfering with the listening of the original sound of the content. Such synthesis of the audio signals may be performed by the content distribution device 30 or by the content playback device 10.

In the first modification, an example is shown in which the content reproducing device 10 performs the modulation process. The content reproducing device 10 may perform modulation processing in accordance with modulation information included in the meta information, or in response to an instruction from a user. As an example, a user may select a modulation setting (e.g., a modulation function or modulation depth). In this case, the content playback device 10 may present information on recommended modulation settings to the user based on the modulation information included in the meta information.

In the second modification, an example has been described in which the content playback device 10 selects the content to be recommended to the user and the mode in which the content is to be played back, and automatically controls the playback of the content in accordance with the selection result. However, the content or mode selected by the content playback device 10 may be changed by the user. This allows the user to decide the content to actually view or the mode in which to view the content based on his or her own will or preference, while referring to the selection result by the content playback device 10 as a basis for making a decision.

Although the embodiments of the present invention have been described in detail above, the scope of the present invention is not limited to the above-described embodiments. Furthermore, the above-described embodiment can be improved or modified in various ways without departing from the spirit and scope of the present invention. Furthermore, the above-described embodiments and modifications can be combined.

### [Reference Signs List]

1: Cognitive function improvement system
10: Content playback device
11: Storage device
12: Processor
13: Input/Output Interface
14: Communication interface
21: Display
22: Sound output device
30: Content distribution device
31: Storage device
32: Processor
33: Input/Output Interface
34: Communication interface
41: Sound source device

## Claims

1. A signal processing apparatus comprising:
a receiving means for receiving an input audio signal;
a generating means for generating a modulated audio signal having an amplitude change corresponding to a frequency of gamma waves by performing a modulation process including amplitude modulation on the input audio signal received by the receiving means; and
an output means for outputting a content and modulation information in association with each other, the content including the modulated audio signal generated by the generation means, the modulation information being related to the modulation process performed to generate the modulated audio signal.

2. The signal processing apparatus according to claim 1, further comprising
a reference value acquisition means for acquiring a reference value of a score determined based on
a modulation process, wherein
the generating means performs a modulation process on the input audio signal so that a score determined based on the modulation process performed to generate the modulated audio signal from the input audio signal received by the receiving means is equal to or greater than the reference value acquired by the reference value acquiring means.

3. A signal processing apparatus comprising:
a modulation information acquisition means for acquiring modulation information associated with a modulated audio signal having an amplitude change corresponding to a frequency of gamma waves, the modulation information being related to a modulation process including amplitude modulation performed to generate the modulated audio signal; and
a control means for controlling, based on the modulation information, playback of a content including the modulated audio signal associated with the modulation information acquired by the modulation information acquisition means.

4. The signal processing apparatus according to claim 3, wherein
the control by the control means includes control for displaying, on a screen displaying an image included in the content to be played back, the modulation information related to the modulation process performed to generate the modulated audio signal included in the content.

5. The signal processing apparatus according to claim 3, further comprising
a user information acquisition means for acquiring user information related to at least one of a user's attribute, an operation history, or a viewing history, wherein
the control by the control means includes control of prompting a user to play a content that is determined based on the user information acquired by the user information acquisition means and the modulation information acquired by the modulation information acquisition means.

6. The signal processing apparatus according to claim 3, further comprising
a reference value acquisition means for acquiring a reference value of a score determined based on a modulation process, wherein
the control by the control means includes control for generating a list of one or more contents to be played back so that a sum of scores determined based on the modulation process performed to generate modulated audio signals included in the contents in the list is equal to or greater than the reference value acquired by the reference value acquisition means.

7. The signal processing apparatus according to any one of claims 1 to 6, wherein
the modulation information includes at least one of information indicating that a modulation process has been performed, information indicating the content of the modulation process, or information indicating a score determined based on the modulation process.

8. The signal processing apparatus according to any one of claims 1 to 6, wherein
the modulated audio signal has an amplitude change corresponding to a frequency between 35 Hz and 45 Hz.

9. Cognitive function improvement system comprising a content distribution apparatus and a content playback apparatus, wherein
the content distribution apparatus comprises:
a receiving means for receiving an input audio signal;
a generating means for generating a modulated audio signal having an amplitude change corresponding to a frequency of gamma waves by performing a modulation process including amplitude modulation on the input audio signal received by the receiving means; and
an output means for outputting a content and modulation information in association with each other, the content including the modulated audio signal generated by the generation means, the modulation information being related to the modulation process performed to generate the modulated audio signal, and wherein
the content playback apparatus comprises:
a modulation information acquisition means for acquiring the modulation information output by the output means; and
a control means for controlling playback of the content output by the output means, based on the modulation information acquired by the modulation information acquisition means.

10. A signal processing method comprising:
receiving an input audio signal;
generating a modulated audio signal having an amplitude change corresponding to a frequency of gamma waves by performing a modulation process including amplitude modulation on the received input audio signal; and
outputting a content and modulation information in association with each other, the content including the modulated audio signal generated, the modulation information being related to the modulation process performed to generate the modulated audio signal.

11. A signal processing method comprising:
acquiring modulation information associated with a modulated audio signal having an amplitude change corresponding to a frequency of gamma waves, the modulation information being related to a modulation process including amplitude modulation performed to generate the modulated audio signal; and
controlling, based on the modulation information, playback of a content including the modulated audio signal associated with the acquired modulation information.

12. A program for causing a computer to execute the signal processing method according to claim 10 or 11.
